# EUROPÄISCHE PATENTANMELDUNG

(11) **EP 2 832 716 A1**
(43) Veröffentlichungstag der Anmeldung: **04.02.2015**
(21) Anmeldenummer: 13178346.6
(22) Anmeldetag: 29.07.2013
(51) Int. Cl.: C07C 5/333, C07C 5/48, C07C 11/167

(54) **1,3-Butadien-Synthese**

(71) Anmelder: LANXESS Deutschland GmbH, 50569 Köln (DE)
(72) Erfinder: Haufe, Rommy, 57439 Attendorn (DE); Föllinger, Thomas, 41539 Dormagen (DE)

(57) **Zusammenfassung**

Die Erfindung betrifft ein zweistufiges Verfahren zur selektiven Herstellung von 1,3-Butadien aus n-Butan.

## Beschreibung

Die Erfindung betrifft ein Verfahren zur selektiven Herstellung von 1,3-Butadien aus n-Butan.

Butadien kann durch thermische Spaltung (Steam-Cracken) gesättigter Kohlenwasserstoffe hergestellt werden, wobei üblicherweise von Naphtha oder Kohlenwasserstoffen im Bereich C2 bis C4 als Rohstoff ausgegangen wird.

Beim Aufarbeiten des beim Cracken gebildeten Stoffgemisches fällt ein sogenannter C4-Schnitt an, der neben 1,3-Butadien auch 1,2-Butadien, n-Butan, Butene, 1-Butin, Butenin und Propin enthält.

Nachteilig an der Gewinnung von 1,3-Butadien aus Crackerprodukten ist, dass als Crackerrohstoff zu Lasten des Naphtha immer mehr Ethan eingesetzt wird. Das Cracken von Ethan liefert aber nur minimale Mengen an 1,3-Butadien, so dass dieses stetig knapper und teurer wird.

Aus der WO 2006/075025 A1 ist ein Verfahren zur Herstellung von Butadien aus n-Butan bekannt. Ein n-Butan enthaltender Einsatzgasstrom wird bei diesem Verfahren in mindestens eine erste Dehydrierzone eingespeist und darin nicht-oxidativ katalytisch dehydriert. Der resultierende Produktgasstrom aus der nicht-oxidativen, katalytischen Dehydrierung wird zusammen mit sauerstoffhaltigem Gas einer zweiten Dehydrierungszone zugeführt und darin oxidativ dehydriert um einen Produktgasstrom zu erhalten, der gegenüber dem ersten Produktgasstrom einen höheren Gehalt an Butadien aufweist. Daran schließt sich die Abtrennung der leichtsiedenden Nebenbestandteile vom C₄-Gasstrom an, wobei der C₄-Gasstrom einer Extraktivdestillation unterzogen wird und der aus n-Butan und 2-Buten bestehende Gasstrom in die erste Dehydrierzone rückgeführt wird.

Nachteilig am Verfahren aus WO 2006/075025 A1 sind die hohen Rückführströme sowie die im ersten Reaktionsschritt unbefriedigende Ausbeute an Buten /Butadien infolge der geringen Umsetzung des Butans von weniger als 50%. Gemäß WO 2006/075025 A1 enthält der Strom 5 immer noch 0,3340 kg/h Butan, wenn im Strom 4 zuvor 0,6716 kg/h Butan eingesetzt wurden.

Aufgabe der Erfindung ist es, ein Verfahren zur Herstellung von 1,3-Butadien aus n-Butan bereitzustellen, bei dem in möglichst geringem Umfang Koppelprodukte, wie insbesondere 1,2-Butadien, anfallen, hohe Rückführströme vermieden werden und die Ausbeute an 1,3-Butadien bereits in der ersten Stufe möglichst hoch ist.

Gelöst wird die Aufgabe durch ein Verfahren zur Herstellung von 1,3-Butadien **dadurch gekennzeichnet, dass** man
A) einen n-Butan enthaltenden Einsatzgasstrom in einer ersten Stufe in einem beheizten Röhrenreaktor unter Einsatz eines Katalysators X bei Temperaturen im Bereich von 450 bis 900 ° C dehydriert und
B) das Reaktionsgemisch aus A) unter Zusatz eines sauerstoffhaltigen Gases in einem Dehydrierungsreaktor bei Temperaturen im Bereich von 450 bis 950 ° C umsetzt,
   unter Einsatz wenigstens eines Katalysators X in Verfahrensschritt A) enthaltend
   a) einen Formkörper aus
      i) mindestens einem oder mehreren Oxiden von Elementen der II. bis IV. Haupt- oder Nebengruppe des Periodensystems oder einer daraus aufgebauten oxidischen Mischverbindung; wobei der/die Bestandteil(e) als Basismaterial des Formkörpers dienen, und
      ii) wenigstens einer Zusatzkomponente eines Oxids eines Elementes der IV. Hauptgruppe des Periodensystems,
   b) einer auf den Formkörper aufzubringenden aktiven Oberflächenkomponente, die wenigstens eine Platinkomponente beinhaltet und
   c) einer auf den Formkörper zusätzlich aufzubringenden Oberflächenkomponente einer Verbindung eines Elementes der III. oder IV. Hauptgruppe des Periodensystems,
und unter Einsatz in Verfahrensschritt B) wenigstens eines Katalysators X sowie wenigstens eines Katalysators Y wobei der Katalysator Y ein poröser Körper der Sauerstoff spendet ist und wenigstens ein Oxid oder eine Mischung von Oxiden ausgewählt aus der Reihe MgO, MoO, MoO₂ MoOs Bi₂O₃, Bi₂O₅, CoO, Co₂O₃, Co₃O₄, NiO, NiAl₂O₄, CuO, Mn₃O₄, Fe₂O₃, Fe₃O₄, FeO, TiO₂ und SiO₂ enthält.

Zur Klarstellung sei angemerkt, dass vom Rahmen dieser Erfindung alle nachfolgend aufgeführten allgemeinen oder in Vorzugsbereichen genannten Definitionen und Parameter in beliebigen Kombinationen umfasst sind.

Im Dehydrierungsreaktor des Verfahrensschrittes B) wird zusätzlich ein sauerstoffhaltiges Gas dem Einsatzgemisch zugeführt, um den während der Dehydrierung gebildeten Wasserstoff zur Oxidation zu bringen und die Reaktion der Dehydrierung in die gewünschte Richtung zu verschieben. Wenn der insgesamt benötigte Sauerstoff nur im Eingangsbereich des in Schritt B) einzusetzenden Dehydrierungsreaktors zugeführt wird, kommt in den vom Eingangsbereich weiter entfernten Teilen des Katalysatorbetts des in Verfahrensschritt B) einzusetzenden Dehydrierungsreaktors der erforderliche Sauerstoff nicht in jedem Falle wie gewünscht an. Andererseits führt zu viel Sauerstoff im Eingangsbereich des in Verfahrensschritt B) einzusetzenden Dehydrierungsreaktors zur Beschädigung des Katalysators im vom Eingangsbereich weiter entfernten Bereichen des Katalysatorbetts. In einer Ausführungsform der vorliegenden Erfindung wird deshalb der insgesamt benötigte Sauerstoff an mehreren Einspeisestellen zuzugeben, die entlang des Katalysatorbettes angeordnet sind.

Die in Verfahrensschritt B) insgesamt zugeführte Menge des sauerstoffhaltigen Gases wird bevorzugt so gewählt, dass ein molares Sauerstoff : n-Buten-Verhältnis von mindestens 0,5 entsteht. Besonders bevorzugt wird bei einem molaren Sauerstoff : n-Buten-Verhältnis von 0,55 bis 50 gearbeitet.

Ein einer bevorzugten Ausführungsform wird in Verfahrensschritt A) ein beheizter Röhrenreaktor eingesetzt.

Erfindungsgemäß bevorzugt wird in Verfahrensschritt B) ein Dehydrierungsreaktor ohne Beheizung eingesetzt.

Beheizte im Verfahrensschritt A) einzusetzende Röhrenreaktoren, insbesondere Rohrbündelreaktoren, sind beispielsweise in DE 10237514 A1, DE 10 2004 040 472 A1, DE 10 2005 001 952 A1, DE 20 2006 014 116 U1, DE 10 2007 061 477 A1 oder in WO 2010/083978 AA2 beschrieben und werden beispielsweise von Thyssen Krupp Uhde GmbH, Dortmund, Deutschland hergestellt.

Im Verfahrensschritt B) einzusetzende Dehydrierungsreaktoren ohne Beheizung oder Kühlung sind beispielsweise in "Verfahrenstechnische Berechnungen - Teil 5: Chemische Reaktoren", VCH Verlagsgesellschaft, Weinheim 1987 beschrieben und werden ebenfalls von ThyssenKrupp Uhde GmbH hergestellt.

Das erfindungsgemäße Verfahren zeichnet sich durch eine gegenüber dem Stand der Technik verbesserte Selektivität und damit besonders hohe 1,3-Butadien Ausbeute aus. Durch die besondere Kombination von Reaktortyp und Katalysator werden Verluste des Rohstoffs n-Butan durch Rückführung von nicht umgesetztem n-Butan in die Dehydrierung minimiert.

Für den ersten Verfahrensschritt A) wird ein n-Butan enthaltender Einsatzgasstrom bereitgestellt. Üblicherweise wird dieser ausgehend von n-Butan reichen Gasgemischen wie liquefied petroleum gas (LPG) als Rohstoff durchgeführt. LPG enthält im Wesentlichen gesättigte C₂-C₅-Kohlenwasserstoffe. Daneben enthält es auch Methan und Spuren von Kohlenwasserstoffen mit sechs und mehr Kohlenstoffatomen. Die Zusammensetzung von LPG kann stark schwanken. Vorteilhafterweise enthält das eingesetzte LPG mindestens 10 Gew.% Butane. Alternativ kann ein veredelter C₄-Strom aus Crackern oder Raffinerien eingesetzt werden.

In einer bevorzugten Ausführungsform enthält der n-Butan enthaltende Einsatzgasstrom mindestens 60 Gew.-% n-Butan, besonders bevorzugt mindestens 90 Gew.-% n-Butan. Daneben kann er als Nebenbestandteile noch weitere C₁-C₄-Kohlenwasserstoffe enthalten.

Im Verfahrensschritt A) wird der n-Butan enthaltende Einsatzgasstrom in eine Dehydrierzone eines Röhrenreaktors, bevorzugt eines Rohrbündelreaktors, eingespeist und einer nicht-oxidativen katalytischen Dehydrierung unterworfen. In diesem als Dehydrierungsreaktor arbeitenden Röhrenreaktor wird n-Butan an einem dehydrieraktiven Katalysator X teilweise zu 1-Buten und 2-Buten dehydriert, wobei auch 1,3-Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO,CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

In einer Ausführungsform der vorliegenden Erfindung kann die nicht-oxidative katalytische n-Butan Dehydrierung im Verfahrensschritt A) mit oder ohne sauerstoffhaltigem Gas als Co-Feed durchgeführt werden. Vorzugsweise wird sie als nicht-oxidative katalytische Dehydrierung ohne externe Beheizung unter Einspeisung von Sauerstoff als Co-Feed durchgeführt. Bei dieser Fahrweise wird die benötigte Wärme direkt im Reaktorsystem durch Verbrennung von Wasserstoff und/oder Kohlenwasserstoffen in Gegenwart von Sauerstoff erzeugt. In einer bevorzugten Ausführungsform wird zusätzlich ein Wasserstoff enthaltender Co-Feed zugemischt. Sauerstoff kann als Rein-Sauerstoff oder als sauerstoffhaltiges Gas eingespeist werden. Bevorzugtes sauerstoffhaltiges Gas ist Luft oder mit Sauerstoff angereicherte Luft mit einem Sauerstoffgehalt bis 70 Vol.-%. Um den Inertgasanteil zu begrenzen, kann Sauerstoff auch als sauerstoffreiches Gas, vorzugsweise mit einem Sauerstoffgehalt von mindestens 75 Vol.-% oder alternativ mindestens 90 Vol.-% eingespeist werden.

Ein Merkmal der nicht-oxidativen Fahrweise gegenüber einer oxidativen Fahrweise ist, dass bei der oxidativen Dehydrierung kein freier Wasserstoff gebildet wird.

Erfindungsgemäß wird im Verfahrensschritt A) ein Röhrenreaktor eingesetzt. Bei diesem Reaktortyp befindet sich der Katalysator X (Dehydrierungskatalysator und, bei Arbeiten mit Sauerstoff als Co-Feed, gegebenenfalls spezieller Oxidationskatalysator) als Festbett in einem Reaktionsrohr oder in mehreren parallel angeordneten Reaktionsrohren. Die Reaktionsrohre werden üblicherweise indirekt beheizt, indem im die Reaktionsrohre umgebenden Raum ein Brennstoff, bevorzugt ein Kohlenwasserstoff, besonders bevorzugt Methan, verbrannt wird. Günstig und deshalb bevorzugt ist es dabei, diese indirekte Form der Aufheizung lediglich auf den ersten ca. 20 bis 30% der Länge der Katalysator-Festbettschüttung im Röhrenreaktor anzuwenden.

Übliche Reaktionsrohr-Innendurchmesser betragen etwa 10-15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 450 bis 900 °C, vorzugsweise im Bereich von 500 bis 650 °C.

In einer Ausführungsform kann Verfahrensschritt A) unter Verwendung einer geringen Wasserdampfverdünnung durchgeführt werden. In diesem Fall liegt der Arbeitsdruck liegt in diesem Fall üblicherweise im Bereich von 0.5 bis 8 bar, bevorzugt im Bereich von 1 bis 2 bar (analog dem Linde-Verfahren zur Propan-Dehydrierung).

Im Falle einer hohen Wasserdampfverdünnung kann der Arbeitsdruck aber auch im Bereich von 3 bis 8 bar liegen(analog dem sogenannten "steam active reforming process" (STAR-Prozess) zur Dehydrierung von Propan oder Butan von Phillips Petroleum Co., siehe US 4,902,849, US 4,996,387 und US5,389,342). Typische und bevorzugte Katalysatorbelastungen (GHSV) liegen im Bereich von 500 bis 1000 h⁻¹, bezogen auf das eingesetzte n-Butan. Die Katalysatorgeometrie kann beispielsweise kugelförmig oder zylindrisch (hohl oder voll) sein.

Wird die nicht-oxidative katalytische n-Butan Dehydrierung in Verfahrensschritt A) mit oder ohne sauerstoffhaltigem Gas als Co-Feed durchgeführt, so wird die Menge des dem Reaktionsgasgemisch zugesetzten sauerstoffhaltigen Gases so gewählt, dass durch die Verbrennung von im Reaktionsgasgemisch vorhandenem Wasserstoff und gegebenenfalls von im Reaktionsgasgemisch vorliegenden Kohlenwasserstoffen und/oder in Form von Koks vorliegendem Kohlenstoff die für die Dehydrierung des n-Butans benötigte Wärmemenge erzeugt wird.

Bevorzugt beträgt die in Verfahrensschritt A) insgesamt zugeführte Sauerstoffmenge, bezogen auf die Gesamtmenge des n-Butans, 0,001 bis 0,5 mol/mol, besonders bevorzugt 0,005 bis 0,25 mol/mol, ganz besonders bevorzugt 0,05 bis 0,25 mol/mol. Sauerstoff kann entweder als reiner Sauerstoff oder als sauerstoffhaltiges Gas im Gemisch mit Inertgasen, bevorzugt in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan Dehydrierung gebildete Wasserstoff sowie gegebenenfalls dem Reaktionsgemisch als wasserstoffhaltiges Gas zusätzlich zugesetzter Wasserstoff. Vorzugsweise sollte so viel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Katalysator X katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist.

In Verfahrensschritt B) erfolgt die Dehydrierung des aus Verfahrensschritt A) erhaltenen Gasstroms in Gegenwart wenigstens eines Katalysators X sowie wenigstens eines Katalysators Y in einem Dehydrierungsreaktor ohne Beheizung unter Zusatz eines sauerstoffhaltigen Gases, wobei der Katalysator Y selektiv die Verbrennung von Wasserstoff mit Sauerstoff in Gegenwart von Kohlenwasserstoffen katalysiert. Die Verbrennung dieser Kohlenwasserstoffe mit Sauerstoff zu CO, CO₂ und Wasser läuft dadurch nur in untergeordnetem Maße ab. In einer Ausführungsform liegen der im Verfahrensschritt B) wenigstens eine einzusetzende Katalysator X und der wenigstens eine einzusetzende Katalysator Y in verschiedenen Reaktionszonen des Dehydrierungsreaktors vor.

Bei mehrstufiger Reaktionsführung kann der wenigstens eine Katalysator Y in nur einer, in mehreren oder in allen Reaktionszonen des nicht beheizten Dehydrierungsreaktors des Verfahrensschrittes B) vorliegen.

Bevorzugt ist der wenigstens eine Katalysator Y, der selektiv die Oxidation von Wasserstoff katalysiert, an den Stellen des Dehydrierungsreaktors des Verfahrensschritt B) angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung desselben und/oder des kohlenwasserstoffhaltigen Gases kann an einer oder an mehreren Stellen des Dehydrierungsreaktors erfolgen.

Das Reaktionsgemisch aus Verfahrensschritt A) wird im Verfahrensschritt B) unter Zusatz eines sauerstoffhaltigen Gases in einem Dehydrierungsreaktor in Anwesenheit sowohl wenigstens eines Katalysators X als auch wenigstens eines Katalysators Y bei Temperaturen im Bereich von 450 bis 950 ° C umgesetzt, bevorzugt bei Temperaturen im Bereich von 580 bis 700 ° C. Erfindungsgemäß handelt es sich bei dem Katalysator X zur Durchführung einer katalytischen n-Butan Dehydrierung um einen Katalysator basierend auf einem Formkörper. Der Formkörper enthält i) mindestens ein oder mehrere Oxid(e) von Elementen der II. bis IV. Haupt- oder Nebengruppe des Periodensystems oder einer daraus aufgebauten oxidischen Mischverbindung sowie zusätzlich wenigstens eine Zusatzkomponente ii) eines Oxids eines Elementes der IV. Hauptgruppe des Periodensystems. Dieses Oxid wird typischerweise während des Formgebungsprozesses des Formkörpers hinzugegeben. Die Verbindung(en) (i) dient/dienen als Basismaterial des Formkörpers. Bevorzugt beträgt der Gehalt der Basismaterialien (i) mehr als 90 Gew.-% der Bestandteile des Katalysators, besonders bevorzugt liegt der Gehalt der Basismaterialien im Bereich von 96 bis 99,9 Gew.-% der Bestandteile des Katalysators. Der Anteil der Zusatzkomponente ii), ausgewählt aus einem Oxid eines Elementes der IV. Hauptgruppe des Periodensystems, beträgt bevorzugt 0,1 bis 10 Gew. % des Gehalts an Basismaterialien im Katalysator X .

Der Anteil der beiden Oberflächenkomponenten b) und c) liegt bevorzugt im Bereich von 0.1 bis 4 Gew.-% des Katalysators X und wird während des Formgebungsprozesses hinzugegeben. Die Summe aller Komponenten im Katalysator X beträgt stets 100 Gew.-%.

Weiterhin enthält der wenigstens eine erfindungsgemäß in Verfahrensschritt A) bzw. auch in Verfahrensschritt B) einzusetzende Katalysator X b) eine katalytisch wirkende Oberflächenkomponente aus einer Platinkomponente sowie c) eine auf den Formkörper aufzubringende Oberflächenkomponente in Form einer Verbindung eines Elementes der III. oder IV. Hauptgruppe des Periodensystems. Eine Platinkomponente im Sinne der vorliegenden Erfindung bedeutet elementares Platin oder ein Platinlegierung. Gegebenenfalls werden zur Herstellung des Katalysators X weitere Oxide als Komponente iii) eingesetzt, wobei in diesem Fall die Gewichtsprozente der Komponenten i) und ii) sowie der katalytisch wirkenden Oberflächenkomponenten reduziert werden, dass die Summe aller Gewichtsprozente im Katalysator stets 100 ergibt.

Ein bevorzugtes Basismaterial i) des Formkörpers für den Katalysator X wird aus der Reihe Aluminiumoxid, Calciumoxid, Zinkoxid, Zirkoniumdioxid, Magnesiumdioxid und Siliziumdioxid als Hauptbestandteil ausgewählt. Dieses Basismaterial ist üblicherweise mit mehr als 50% der überwiegende Mengenbestandteil des Katalysators X. Das Formkörpermaterial kann auch aus Mischphasen von ausgewählten Stoffen der vorgenannten Liste bestehen. Natürlich kann eine Kombination der Stoffe innerhalb des oben beanspruchten Rahmens auch als Formkörpermaterial eingesetzt werden. Ein bevorzugtes Basismaterial i) des Formkörpers für den Katalysator X zur erfindungsgemäßen Dehydrierung von n-Butan ist Zinkoxid mit Aluminiumoxid, auch als Zinkaluminat bezeichnet. Dieses Zinkaluminat kann beispielsweise durch einen Kalzinierungsprozess aus Zinkoxid und Aluminiumoxid in einem Hochtemperaturofen hergestellt werden, es kann aber auch durch eine Fällungsreaktion einer wässrigen oder alkoholischen Mischung aus einer Zinksalzlösung mit einer Aluminiumsalzlösung hergestellt werden.

Als Zusatzkomponente ii), ausgewählt aus einem Oxid eines Elementes der IV. Hauptgruppe des Periodensystems, wird Zinkdioxid bevorzugt. Obwohl die Zusatzkomponente ii) eine geringe Konzentration im Formkörper aufweist, lässt sie sich jedoch bei der Röntgenbeugung mit der Wellenlänge Cu-K_{α} durch die charakteristischen Reflektionswinkel 26,6°, 33,8° und 51,7° erkennbar nachweisen.

Die katalytisch wirkenden Oberflächenkomponenten b) und c) auf dem Formkörper des Katalysators X erhöhen zusätzlich die Aktivität und die Standzeit dieses Katalysators im Betrieb, wobei einerseits die Platinkomponente b) mit 0,01 bis 1,0 Gew.-% an Platin und andererseits die Oberflächenkomponente c), einem Element der III. oder IV. Hauptgruppe des Periodensystems, bevorzugt Ge, Ga, In oder Sn, besonders bevorzugt Sn als einem Element der IV. Hauptgruppe des Periodensystems, ganz besonders bevorzugt Sn mit einem Anteil von 0,1 bis 4,0 Gew.-% eingesetzt wird. Für die Herstellung des Katalysators X zur erfindungsgemäßen Dehydrierung von n-Butan in Verfahrensschritt A) wird der Formkörper des Katalysators in einem oder mehreren Imprägnierschritten simultan oder konsekutiv mit der aktiven Oberflächenkomponente b) und der zusätzlichen Oberflächenkomponente c) imprägniert und der so hergestellte Formkörper in weiteren Verfahrensschritten zum einsatzbereiten Katalysator X aufgearbeitet.

In einer typischen Ausführungsform werden die festen Rohstoffe des Hauptbestandteils des Formkörpers aus i) mindestens einem oder mehreren Oxiden von Elementen der II. bis IV. Haupt- oder Nebengruppe des Periodensystems oder einer daraus aufgebauten oxidischen Mischverbindung, und einem geringen Anteil der Zusatzkomponente ii), nämlich einem Oxid eines Elementes der IV. Hauptgruppe des Periodensystems, gemahlen, gegebenenfalls mit Bindemittel als Komponente iii) vermischt und im Formgebungsprozess zum Formkörper gefertigt. Hierzu geeignete Formgebungsprozesse sind bevorzugt Sintern, Pelletieren, Tablettieren, Prillen oder Extrusionprozesse.

Nach dem Formgebungsprozess muss der Formkörper kalziniert oder getrocknet werden. Erst danach können die aktiven und zusätzlichen katalytischen Oberflächenkomponenten c) und d) auf den Formkörper, bevorzugt mittels Imprägnierung, Fällung oder Tränkung, insbesondere in Form von Salz in wässriger Lösung, simultan oder konsekutiv aufgetragen werden. In einer Ausführungsform können die Verfahrensschritte des Aufbringens der Oberflächenkomponenten b) und c) wiederholt werden.

In einer bevorzugten Ausgestaltung des Verfahrens zur Herstellung des Katalysators X wird eine oxidische Verbindung für den Formkörper bevorzugt. Besonders bevorzugt sind ein oder mehrere Stoffe aus der Gruppe Zinnoxid, Aluminiumoxid, Calciumoxid, Zirkoniumdioxid, Zinkoxid, Siliziumdioxid, Magnesiumoxid. Die Feststoffe der oxidischen Verbindungen werden pulverisiert, mit Bindemitteln vermischt und einem Formgebungsprozess unterzogen. Weitere begünstigte Varianten für den Formkörper sind auch ein wasserlösliches Zinnsalz und ein oder mehrere wasserlösliche Salze der Metalle Aluminium, Zink, Calcium oder Magnesium. Die wässrigen oder alkoholischen Lösungen werden gegebenenfalls mit entionisiertem Wasser vermischt, neutralisiert und gefällt. Nach der Fällung wird das so gewonnene Material filtriert, getrocknet und durch einen geeigneten Formgebungsprozess zum gewünschten Formkörper verarbeitet. Besonders geeignete Formgebungsprozesse sind typischerweise die Tablettierung oder Extrusion. Die Art des Formgebungsprozesses bleibt dem Fachmann überlassen. Ziel ist es üblicherweise, einen abriebfesten Formkörper mit genügend großer Porosität zu erhalten.

In einer bevorzugten Ausführungsform wird der Formkörper für den Katalysator X mit weiteren katalytisch aktiven Materialien behandelt. Eine zur Aufbringung der Platinkomponente b) auf den Formkörper zum Imprägnieren besonders geeignete Platinverbindung ist Hexachloroplatin(IV)säure oder deren Salze sowie andere lösliche Platinverbindungen, insbesondere Platin(II)halogenide, Platin(IV)halogenide, Platin(II)nitrat, wobei Halogenid bevorzugt für Fluorid, Chlorid, Bromid oder Jodid steht, insbesondere für Chlorid. Für die Imprägnierung mit der zusätzlichen Oberflächenkomponente c), einer Verbindung eines Elementes der III. oder IV. Hauptgruppe des Periodensystems, wird bevorzugt eine wasserlösliche Zinnverbindung als Vertreter der IV. Hauptgruppe des Periodensystems eingesetzt, besonders bevorzugt Zinnchlorid oder Zinnnitrat. Für das Imprägnieren kann sowohl eine wässrige als auch eine ethanolische oder methanolische Lösung der jeweiligen Oberflächenkomponente b) oder c) eingesetzt werden. Das Imprägnieren des Formkörpers mit den angegebenen Oberflächenkomponenten b) und/oder c) in Lösungen kann konsekutiv oder simultan erfolgen und gegebenenfalls mehrfach wiederholt werden.

Das Imprägnieren erfolgt typischerweise durch Aufsprühen oder Tränken des Formkörpers mit der die Oberfächenkomponenten b) und c) enthaltenden Lösung(en). Im Prinzip sind auch andere Verfahren als Imprägnierverfahren geeignet, welche eine gleichmäßige Verteilung der zu imprägnierenden Oberflächenkomponenten b) und c) auf den Formkörper gewährleisten.

Nach der Imprägnierung durchläuft der imprägnierte Formkörper je nach Bedarf die nachfolgenden Verfahrensschritte Kalzinieren, Waschen und/oder Trocknen und Reduzieren. Einzelne dieser Verfahrensschritte können auch wiederholt werden. Danach ist der Katalysator X einsatzbereit.

Besonders bevorzugt wird als Katalysator X ein Magnesiumaluminat-, Zinkaluminat- oder Calciumaluminat-Träger mit Platin-, Zinn- oder Indium-Imprägnierung eingesetzt.

Besonders bevorzugt wird ein Magnesiumaluminat-, Zinkaluminat- oder Calciumaluminat-geträgerter PtSn, PtGa oder PtIn Katalysator, insbesondere PtSn, als Katalysator X eingesetzt.

Ganz besonders bevorzugt wird als Katalysator X ein trimetallischer Magnesiumaluminat-, Zinkaluminat- oder Calciumaluminat-geträgerter PtSnIn Katalysator oder ein PtSnGa Katalysator eingesetzt. Insbesondere bevorzugt wird ein PtSnGa Katalysator oder PtSnIn Katalysator auf einem MgAl₂O₄ -Träger als Katalysator X eingesetzt.

In Verfahrensschritt B) wird zusätzlich zum wenigstens einen Katalysator X wenigstens ein Katalysator Y eingesetzt. Für den wenigstens einen Katalysator Y wird ein poröser Körper, der Sauerstoff spendet, eingesetzt. Es gibt viele unterschiedliche Sauerstoffspender von Metalloxiden. Aber unter den vorgegebenen oxidativen Dehydrierungsbedingungen wird als Katalysator Y wenigstens ein Oxid oder eine Mischung von Oxiden der Reihe MgO, MoO, MoO₂, MoO₃ Bi₂O₃, Bi₂O₅, CoO, Co₂O₃, Co₃O₄, NiO, NiAl₂O₄, CuO, Mn₃O₄, Fe₂O₃, Fe₃O₄, FeO, TiO₂, eingesetzt.

Besonders bevorzugt wird in Verfahrensschritt B) als Katalysator Y eine Kombination aus Zn-, Mg-, und Mn-Ferriten, insbesondere ZnFe₂O₄, MnFe₂O₄, MgFe₂O₄ und/ oder Mo-Bi-Mischoxiden eingesetzt; entweder physikalisch gemischt oder in Form wenigstens einer hintereinandergeschalteten Katalysatorschüttung. Physikalisch gemischt bedeutet im Sinne der vorliegenden Erfindung nebeneinander vorliegend ohne chemische Wechselwirkung zwischen den Oxiden.

Besonders bevorzugte Mo-Bi-Mischoxide sind Multimetalloxidaktivmassen der allgemeinen Formel (I)

Mo₁₂BiₐFe_{b}W_{c}X_{d}YₑZ_{f}O_{g} (I)

worin
X für Co und/oder Ni steht,
Y für Si und/oder Al steht,
Z für Li, Na, K, Cs und/oder Rb steht,
0,2 ≤ a ≤ 2,
0,5 ≤ b ≤ 10,
0 ≤ c ≤ 4,
2 ≤ d ≤ 10,
0 ≤ e ≤ 10,
0 ≤ f ≤ 0,5, und
g für eine Zahl steht, die unter der Voraussetzung der Ladungsneutralität durch die Wertigkeit und Häufigkeit der von Sauerstoff verschiedenen Elemente in (I) bestimmt wird, oder
Multimetalloxidaktivmassen der Formel (II)

MoₐBi_{b}Co_{c}Ni_{d}FeₑX_{f}Y_{g}ZₕSiᵢOⱼ (II)

worin
X für wenigstens ein Metall der Reihe Sm, Mg, Ca, Zn, Ce steht,
Y für wenigstens ein Metall der Reihe Na, K, Rb, TI, Cs steht,
Z für wenigstens ein Element der Reihe W, As, B, P steht,
und wenn a = 12, dann sind b = 0.5 bis 7, c = 0 bis 10, d = 0 bis 10, e = 0.05 bis 3, f = 0 bis 2, g = 0,04 bis 2, h = 0 bis 3, i = 0 bis 48 (Endwerte eingeschlossen) und j steht für eine Zahl, die unter der Voraussetzung der Ladungsneutralität durch die Wertigkeit und Häufigkeit der von Sauerstoff verschiedenen Elemente in (II) bestimmt wird.

Die Multimetalloxidaktivmassen der Formel (I) sind beispielsweise aus DE 100 63 162 A1 bekannt, ebenso deren Herstellung. Multimetalloxidaktivmassen der Formel (II) werden in JP 2012111699 A beschrieben, Die oben ausgewählten Metalloxide aus MgO, MoO, MoO₂, MoO₃ Bi₂O₃, Bi₂O₅, CoO, Co₂O₃, NiO, NiAl₂O₄, CuO, Mn₃O₄, Fe₂O₃, Fe₃O₄, FeO, TiO₂, SiO₂ sind kostengünstiger als die Metalloxide aus seltenen Erden, und haben hohe Selektivität. Durch die besonderen Strukturen dieser Metalloxide können sie viel Sauerstoff aufnehmen, besitzen hohe Aktivität bei der Reduktion und Oxidation, sind formstabil und abriebfest. Durch diese Eigenschaften sind sie ideale Sauerstoffspender für die Dehydrierung von n-Butan bzw. des aus verfahrensschritt A) erhaltenen Produktgasstroms.

Darüber hinaus wird die Aufgabe der vorliegenden Erfindung auch dadurch gelöst, dass in einer alternativen Ausführungsform die Dehydrierung von n-Butan unter Zumischung von Wasserdampf mit äußerer Wärmezufuhr, bevorzugt bei einem n-Butan-Wasserdampf-Molverhältnis von 1 zu 10, durchgeführt wird. In diesem Fall wird die Dehydrierung bevorzugt bei Temperaturen im Bereich von 450°C bis 900°C, besonders bevorzugt bei Temperaturen im Bereich von 500°C bis 650°C durchgeführt.

Hierbei wird die äußere Wärmezufuhr so variiert, dass die zur Aufrechterhaltung der n-Butan-Umwandlung erforderliche Temperatur eingestellt wird, und die Dehydrierungsumsätze nach dem Starten der Reaktion so lange konstant bleiben, bis die Katalysatoren X und Y regeneriert werden. Im Verfahrensschritt B) gibt der Katalysator Y Sauerstoff ab, der sich mit Wasserstoff, der in der Dehydrierungsreaktion gebildet wird, zu Wasserdampf verbindet, wodurch sich die Dehydrierungsreaktion zur Produktseite - dem 1,3-Butadien - hin verschiebt.

Um die Gleichgewichtsreaktion vollständig zugunsten des 1,3-Butadiens ablaufen zu lassen genügt es, die Menge des n-Butan im Reaktionsgemisch zu erhöhen oder die im Reaktionsprodukt zwangsweise anfallenden Nebenprodukte permanent aus dem Reaktionsgemisch zu entfernen. Die Rückreaktion wird dadurch solange unterbunden, bis das ursprüngliche Gleichgewicht wieder hergestellt ist. Nach der Formel (III) wird die Reaktion der Dehydrierung des n-Butans unter Bildung des 1,3-Butadiens durch Entzug des Produkts (1,3-Butadien) und/oder Wasserstoff (H₂) solange ablaufen, bis die erforderlichen Mengen vom Produkt für das Gleichgewicht wieder verfügbar sind.

C₄H₁₀ ↔ C₄H₆+2H₂ (III)

n-Butan wird mit Wasserdampf über das jeweilige Katalysatorbett geleitet. Dabei wird die Dehydrierungsreaktion durch den ersten Katalysator X beschleunigt. Durch die Dehydrierungsreaktion nach der Formel (III) entsteht 1,3-Butadien und Wasserstoff. Der Wasserstoff entzieht im Verfahrensschritt B) dem dort einzusetzenden Katalysator Y den Sauerstoff, und wird dabei zu Wasserdampf. Durch diese sofort an der Stelle ablaufende Reaktion wird die Rückreaktion der Dehydrierung permanent unterdrückt. Dies führt zu einem hohen stabilen Umsatz der Dehydrierung. Die chemische Reaktion

2H₂ + O₂ → 2H₂O

ist exotherm und setzt dabei 573 kJ pro Mol Wärme frei. Die Temperatur im Reaktor steigt an. Mittels eines Temperaturmessgerätes wird die erforderliche Temperatur zur Aufrechterhaltung der n-Butan-Umwandlung eingestellt, denn die äußere Wärmezufuhr kann variiert werden. Dadurch werden die Energiekosten der Dehydrierungsanlage reduziert.

Außerdem begünstigt Wasserdampf im Verfahrensschritt B) die Vergasung von kohlenstoffhaltigen Ablagerungen auf dem/den Katalysator(en) nach der untenstehenden Formel (IV).

C + 2H₂O ↔ CO₂ + 2H₂ (IV)

Der hier entstandene Wasserstoff verbindet sich wiederum mit dem Sauerstoff aus dem Sauerstoffspender und wird zu Wasserdampf. Dadurch bleiben die Katalysatoren viel länger aktiv. Folglich ist eine länger anhaltende Dehydrierung nach dem Starten der Reaktion zu erwarten, was zu einer verbesserten Wirtschaftlichkeit der Dehydrierungsanlage führt.

Bei der nicht-oxidativen katalytischen n-Butan-Dehydrierung im Röhrenreaktor des Verfahrensschritt A) wird ein Gasgemisch erhalten, das neben 1,3-Butadien 1-Buten, 2-Buten nicht umgesetztes n-Butan sowie Nebenbestandteile enthält. Übliche Nebenbestandteile sind Wasserstoff, Wasserdampf, Stickstoff, CO₂ sowie Leichtsieder, insbesondere Leichtsieder der Reihe Methan, Ethan, Ethen, Propan und Propen. Die Zusammensetzung des den ersten Dehydrierungsreaktor verlassenden Gasgemisches kann abhängig von der Fahrweise der Dehydrierung stark variieren. So weist bei Durchführung der bevorzugten Dehydrierung ohne externe Beheizung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen katalytischen n-Butan-Dehydrierung aus Verfahrensschritt A) enthält typischerweise 0,1 bis 15 Vol.-% 1,3-Butadien, 0 bis 20 Vol.-% 1-Buten, 0 bis 40 Vol.-% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe, insbesondere Methan, Ethan, Ethen, Propan und Propen, 0,1 bis 40 Vol.-% Wasserstoff, 0 bis 70 Vol.-% Stickstoff und 0 bis 15 Vol.-% Kohlenstoffoxide.

Der die erste Dehydrierzone (Verfahrensschritt A) verlassende Produktgasstrom wird in einer Ausführungsform in zwei Teilströme aufgetrennt, wobei nur einer der beiden Teilströme dem weiteren Verfahrensschritt B) unterworfen wird und der zweite Teilstrom in die erste Dehydrierzone zurückgeführt wird. Eine entsprechende Verfahrensweise ist in DE-A 102 11 275 beschrieben. Es kann jedoch auch der gesamte Produktgasstrom der nicht-oxidativen katalytischen n-Butan-Dehydrierung (= Verfahrensschritt A) dem weiteren Verfahrensschritt B) unterworfen werden.

Der nicht-oxidativen katalytischen Dehydrierung im Röhrenreaktor des Verfahrensschritt A) wird erfindungsgemäß eine nicht beheizte oxidative Dehydrierung (Oxidehydrierung) in Verfahrensschritt B) nachgeschaltet. Dabei werden im Wesentlichen 1-Buten und 2-Buten zu 1,3-Butadien dehydriert, wobei 1-Buten im Allgemeinen nahezu vollständig abreagiert.

Die adiabatische, oxidative Dehydrierung in Verfahrensschritt B) kann grundsätzlich in allen aus dem Stand der Technik bekannten Reaktortypen und Fahrweisen durchgeführt werden, bevorzugt im Wirbelbett, im Hordenofen, im Festbettrohr- oder Rohrbündelreaktor oder im Plattenwärmetauscherreaktor. Zur Durchführung der adiabatischen, oxidativen Dehydrierung in Verfahrensschritt B) wird bevorzugt ein Gasgemisch benötigt, welches ein molares Sauerstoff : n-Buten-Verhältnis von mindestens 0,5 aufweist. Besonders bevorzugt wird bei einem molaren Sauerstoff : n-Buten-Verhältnis von 0,55 bis 50 gearbeitet. Zur Einstellung dieses Wertes wird in der Regel das aus der nicht-oxidativen katalytischen Dehydrierung (Verfahrensschritt A)) stammende Produktgasgemisch mit Sauerstoff oder einem sauerstoffhaltigen Gas vermischt. Das sauerstoffhaltige Gas ist wie im Falle der ersten Dehydrierstufe - sofern diese ohne äußere Wärmezufuhr betrieben wird - bevorzugt Luft oder mit Sauerstoff angereicherte Luft mit einem Sauerstoffgehalt bis 70 Vol.-%, vorzugsweise bis 50 Vol.-%. Das erhaltene sauerstoffhaltige Gasgemisch wird dann der Oxidehydrierung in Verfahresschritt B) zugeführt.

Nach einer gewissen Betriebsdauer lässt die Aktivität des Katalysators X im Reaktor des Verfahrensschritt A) durch die darauf abgelagerten Kohlenstoffabscheidungen langsam nach, und der Vorrat an Sauerstoffen aus dem Sauerstoffspender, dem Katalysator Y im adiabatischen Dehydrierungsreaktor des Verfahrensschritt B), neigt sich dem Ende. Wenn der Umsatz der Dehydrierung sinkt, empfielt es sich, die Katalysatoren zu regenerieren.

In einer bevorzugten Ausführungsform schließt sich deshalb an das erfindungsgemäße Verfahren ein Regenerierverfahren an, bei dem die Katalysatoren im jeweiligen Dehydrierungsreaktor regeneriert werden. Das Regenerieren umfasst bevorzugt drei Phasen: Spülphase, Regenerationsphase, Spülphase. Hierbei wird jeweils während einer Spülzeit vor und nach der Regenerationsphase das Katalysatorbett mit Spülmedium durchströmt und während der Regenerationsphase die Katalysatoren mit einem sauerstoffhaltigen und Wasserdampf-haltigen Gas oder einem sauerstoffhaltigen und Inertgas-haltigen Gas so lange behandelt, dass Kohlenstoffabscheidungen zu Kohlendioxid umgesetzt und der Sauerstoff vom entsprechenden Katalysator Y im Dehydrierungsreaktor (für den Verfahrensschritt B)) aufgenommen wird.

Die Katalysatoren werden zum Regenerieren nicht wie in DE 1 442 850 A1 beschrieben, dem Reaktor entnommen sondern sie bleiben im jeweiligen Dehydrierungsreaktor.

Dehydrierungsreaktoren ohne Beheizung sind beispielsweise in "Verfahrenstechnische Berechnungen - Teil 5: Chemische Reaktoren", VCH Verlagsgesellschaft, Weinheim 1987 beschrieben und werden ebenfalls von ThyssenKrupp Uhde GmbH hergestellt.

## Patentansprüche

1. Verfahren zur Herstellung von 1,3-Butadien **dadurch gekennzeichnet, dass** man
A) einen n-Butan enthaltenden Einsatzgasstrom in einer ersten Stufe in einem beheizten Röhrenreaktor unter Einsatz eines Katalysators X bei Temperaturen im Bereich von 450 bis 900 ° C dehydriert und
B) das Reaktionsgemisch aus A) unter Zusatz eines sauerstoffhaltigen Gases in einem Dehydrierungsreaktor bei Temperaturen im Bereich von 450 bis 950 ° C umsetzt,
unter Einsatz wenigstens eines Katalysators X in Verfahrensschritt A) enthaltend
a) einen Formkörper aus
i) mindestens einem oder mehreren Oxiden von Elementen der II. bis IV. Haupt- oder Nebengruppe des Periodensystems oder einer daraus aufgebauten oxidischen Mischverbindung; wobei der/die Bestandteil(e) als Basismaterial des Formkörpers dienen, und
ii) wenigstens einer Zusatzkomponente eines Oxids eines Elementes der IV. Hauptgruppe des Periodensystems,
b) einer auf den Formkörper aufzubringenden aktiven Oberflächenkomponente, die wenigstens eine Platinkomponente beinhaltet, und
c) einer auf den Formkörper zusätzlich aufzubringenden Oberflächenkomponente einer Verbindung eines Elementes der III. oder IV. Hauptgruppe des Periodensystems,
und unter Einsatz in Verfahrensschritt B) wenigstens eines Katalysators X sowie wenigstens eines Katalysators Y wobei der Katalysator Y ein poröser Körper der Sauerstoff spendet ist und wenigstens ein Oxid oder eine Mischung von Oxiden ausgewählt aus der Reihe MgO, MoO, MoO₂, MoO₃ Bi₂O₃, Bi₂O₅, CoO, Co₂O₃, Co₃O₄, NiO, NiAl₂O₄, CuO, Mn₃O₄, Fe₂O₃, Fe₃O₄, FeO, TiO₂ und SiO₂ enthält.

2. Verfahren gemäß Anspruch 1, **dadurch gekennzeichnet, dass** der im Verfahrensschritt B) wenigstens eine einzusetzende Katalysator X und der wenigstens eine einzusetzende Katalysator Y in verschiedenen Reaktionszonen des Dehydrierungsreaktors vorliegen.

3. Verfahren gemäß der Ansprüche 1 oder 2, **dadurch gekennzeichnet, dass** der Dehydrierungsreaktor des Verfahrensschrittes B) ein Hordenofen, ein Festbettrohrreaktor, ein Rohrbündelreaktor oder ein Plattenwärmetauscherreaktor ist.

4. Verfahren gemäß einem der Ansprüche 1 bis 3, **dadurch gekennzeichnet, dass** zur Durchführung der Dehydrierung in Verfahrensschritt B) ein Gasgemisch eingesetzt wird, welches ein molares Sauerstoff : n-Buten-Verhältnis von mindestens 0,5 aufweist bevorzugt ein Sauerstoff : n-Buten-Verhältnis von 0,55 bis 50.

5. Verfahren gemäß einem der Ansprüche 1 bis 4, **dadurch gekennzeichnet, dass** sich ein Regenerierverfahren anschließt, bei dem die Katalysatoren im jeweiligen Dehydrierungsreaktor regeneriert werden.

6. Verfahren gemäß einem der Ansprüche 1 bis 5, **dadurch gekennzeichnet, dass** das Basismaterial des Formkörpers für den Katalysator X aus der Reihe Aluminiumoxid, Calciumoxid, Zinkoxid, Zirkoniumdioxid, Magnesiumdioxid oder Siliziumdioxid und aus Mischphasen dieser Stoffe ausgewählt wird, bevorzugt Zinkoxid mit Aluminiumoxid, auch als Zinkaluminat bezeichnet.

7. Verfahren gemäß einem der Ansprüche 1 bis 6, **dadurch gekennzeichnet, dass** in c) als Element der III. oder IV. Hauptgruppe des Periodensystems Ge, Ga, In oder Sn eingesetzt wird, bevorzugt Sn mit dem Anteil von 0,1 bis 4,0 Massenprozent.

8. Verfahren gemäß Anspruch 7, **dadurch gekennzeichnet, dass** in c) als Element der IV. Hauptgruppe des Periodensystems eine wasserlösliche Zinnverbindung, bevorzugt Zinnchlorid oder Zinnnitrat eingesetzt wird.

9. Verfahren gemäß einem der Ansprüche 1 bis 8, **dadurch gekennzeichnet, dass** die Platinkomponente aus Hexachloroplatin(IV)säure oder deren Salze sowie anderen löslichen Platinverbindungen erzeugt wird.

10. Verfahren gemäß Anspruch 9, **dadurch gekennzeichnet, dass** als andere lösliche Platinverbindungen Platin(II)halogenide, Platin(IV)halogenide, Platin(II)nitrat eingesetzt werden.

11. Verfahren gemäß einem der Ansprüche 1 bis 10, **dadurch gekennzeichnet, dass** in Verfahrensschritt A) als Katalysator X ein Magnesiumaluminat-, Zinkaluminat- oder Calciumaluminat-Träger mit Platin-, Zinn- oder Indium-Imprägnierung eingesetzt wird, bevorzugt ein Magnesiumaluminat-, Zinkaluminat- oder Calciumaluminat-geträgerter PtSn, PtGa oder PtIn Katalysator, insbesondere PtSn.

12. Verfahren gemäß Anspruch 11, **dadurch gekennzeichnet, dass** in Verfahrensschritt A) ein trimetallischer Magnesiumaluminat-, Zinkaluminat- oder Calciumaluminat-geträgerter PtSnIn Katalysator oder ein PtSnGa Katalysator eingesetzt wird, bevorzugt ein PtSnGa Katalysator oder PtSnIn Katalysator auf einem MgAl₂O₄ -Träger.

13. Verfahren gemäß einem der Ansprüche 1 bis 12, **dadurch gekennzeichnet, dass** in Verfahrensschritt B) als Katalysator Y eine Kombination aus Zn- , Mg-, und Mn-Ferriten, insbesondere ZnFe₂O₄, MnFe₂O₄, MgFe₂O₄ und/ oder Mo-Bi-Mischoxiden eingesetzt; entweder physikalisch gemischt oder in Form wenigstens einer hintereinandergeschalteten Katalysatorschüttung eingesetzt wird.

14. Verfahren gemäß Anspruch 13, **dadurch gekennzeichnet, dass** als Mo-Bi-Mischoxide Multimetalloxidaktivmassen der allgemeinen Formel (I)
Mo₁₂BiₐFe_{b}W_{c}X_{d}YₑZ_{f}O_{g} (I)
worin
X für Co und/oder Ni steht,
Y für Si und/oder Al steht,
Z für Li, Na, K, Cs und/oder Rb steht,
0,2 ≤ a ≤ 2,
0,5 ≤ b ≤ 10,
0 ≤ c ≤ 4,
2 ≤ d ≤ 10,
0 ≤ e ≤ 10,
0 ≤ f ≤ 0,5, und
g für eine Zahl steht, die unter der Voraussetzung der Ladungsneutralität durch die Wertigkeit und Häufigkeit der von Sauerstoff verschiedenen Elemente in (I) bestimmt wird, eingesetzt werden.

15. Verfahren gemäß Anspruch 14, **dadurch gekennzeichnet, dass** als Mo-Bi-Mischoxide Multimetalloxidativmassern der Formel (II)
MoₐBi_{b}Co_{c}Ni_{d}FeₑX_{f}Y_{g}ZₕSiᵢOⱼ (II)
worin
X für wenigstens ein Metall der Reihe Sm, Mg, Ca, Zn, Ce steht,
Y für wenigstens ein Metall der Reihe Na, K, Rb, TI, Cs steht,
Z für wenigstens ein Element der Reihe W, As, B, P steht,
und wenn a = 12, dann sind b = 0.5 bis 7, c = 0 bis 10, d = 0 bis 10, e = 0.05 bis 3, f = 0 bis 2, g = 0,04 bis 2, h = 0 bis 3, i = 0 bis 48 (Endwerte eingeschlossen) und j steht für eine Zahl, die unter der Voraussetzung der Ladungsneutralität durch die Wertigkeit und Häufigkeit der von Sauerstoff verschiedenen Elemente in (II) bestimmt wird, eingesetzt werden.

16. Verfahren gemäß einem der Ansprüche 1 bis 15, **dadurch gekennzeichnet, dass** die Dehydrierung von n-Butan unter Zumischung von Wasserdampf mit äußerer Wärmezufuhrdurchgeführt wird.

17. Verfahren gemäß Anspruch 16, **dadurch gekennzeichnet, dass** die Dehydrierung von n-Butan bei einem n-Butan-Wasserdampf-Molverhältnis von 1 zu 10 durchgeführt wird.
